# EUROPÄISCHE PATENTSCHRIFT

(11) **EP 4 217 099 B1**
(45) Veröffentlichungstag und Bekanntmachung des Hinweises auf die Patenterteilung: **11.03.2026**
(21) Anmeldenummer: 21769346.4
(22) Anmeldetag: 19.08.2021
(51) Int. Cl.: B01D 63/02, A61M 1/16

(54) **VORRICHTUNG FÜR DEN STOFFAUSTAUSCH UND VERFAHREN ZU DESSEN HERSTELLUNG**
DEVICE FOR MATERIAL EXCHANGE AND METHOD FOR THE PRODUCTION THEREOF
DISPOSITIF D'ÉCHANGE DE MATIÈRE ET PROCÉDÉ POUR SA PRODUCTION

(30) Priorität: 25.09.2020 DE 102020125108
(43) Veröffentlichungstag der Anmeldung: 02.08.2023
(73) Patentinhaber: enmodes GmbH, 52068 Aachen (DE)
(72) Erfinder: BORCHARDT, Ralf, 52066 Aachen (DE); RITTER, Ilse, Philine, 52064 Aachen (DE); KADEN, Jens, 41751 Viersen (DE)
(74) Vertreter: Cohausz Hannig Borkowski Wißgott
(86) Internationale Anmeldenummer: PCT/EP2021/073007
(87) Internationale Veröffentlichungsnummer: WO 2022/063494

(56) Entgegenhaltungen:
- EP-A2- 0 942 251
- EP-A2- 1 256 372
- WO-A1-01/05449
- JP-A- S62 172 964

## Beschreibung

Die Erfindung betrifft eine Vorrichtung für den Stoffaustausch zwischen einem ersten Medium und einem zweiten Medium umfassend ein Gehäuseelement in dem ein Hohlfaserpaket mit zwischen den axialen Enden des Gehäuseelementes axial erstreckten stoffpermeablen Hohlfasern angeordnet ist, die an ihrem jeweiligen axialen Endbereich zumindest untereinander mit einer Vergussmasse vergossen sind und die von dem ersten Medium umströmbar und vom zweiten Medium durchströmbar sind, wobei das Gehäuseelement, insbesondere auch der Wickelkern an wenigstens einem der axialen Enden, vorzugsweise an beiden axialen Enden, mit einem Deckelelement verschlossen ist.

Das Vergießen mit der Vergussmasse kann nicht nur untereinander zwischen den Hohlfasern erfolgen, sondern vorzugsweise auch zwischen der Gehäuseelementinnenwand und den Hohlfasern, insbesondere auch zwischen einem von den Hohlfasern umgebenen Wickelkern und den Hohlfasern, sofern ein solcher Wickelkern vorhanden ist. Solche Wickelkerne können zum einen vorgesehen sein, um als Trägerelement für die darauf aufzuwickelnden Hohlfasern zu dienen, insbesondere, wenn die Hohlfasern in Form von wenigstens einer Matte um den Wickelkern herumgewickelt sind und andererseits auch die Fluidleitung definieren, z.B. wenn eines der Fluide durch wenigstens einen Kanal im Wickelkern geleitet wird, insbesondere um dieses von außen in den Bereich um die Hohlfasern herum hineinzuleiten oder umgekehrt.

Das benannte Deckelelement kann einen Medienanschluß aufweisen, der mit den axial offenen Hohlfaserenden in Fluidverbindung steht. So besteht die Möglichkeit über das Deckelelement das zweite Medium an die Hohlfasern zuzuführen oder auch von diesen abzuführen. Ein solches Deckelelement ist vorzugsweise an beiden axialen Enden des Gehäuseelementes angeordnet und mit den Randbereichen des Faserbündels und/oder mit dem Gehäuseelement gedichtet verbunden.

Je nach eingesetztem Vergussmaterial kann es zu Randundichtigkeiten kommen, z.B. wenn das Vergussmaterial nicht stoffschließend mit der Gehäuseinnenwand verbunden ist, wie es z.B. bei Silikon der Fall sein kann. Eine Dichtung zwischen dem am Endbereich vergossenen Hohlfaserpaket / Hohlfaserwickel und dem Gehäuse und/oder Deckelelement kann sodann bislang üblicherweise durch einen Dichtungsring erfolgen, der in eine Ringaufnahmenut in der Stirnfläche des Gehäuseelementes oder des Deckelelementes eingesetzt ist.

Das Vergießen und das Dichten stellen somit bei bisherigen Vorrichtungen für den Stoffaustausch zwei separate Maßnahmen dar, die typischerweise nacheinander durchgeführt werden und die Herstellung aufwändig gestalten. Des Weiteren kann die Ringnut in sich eine den Dichtring überbrückende Undichtigkeit aufweisen sowie auch Totraumbereich bilden, in denen ein Fluid stagnieren kann.

Ein Gehäuseelement ist vorzugsweise ein axial erstrecktes rohrförmiges Element, insbesondere mit kreisförmigem freien Innenquerschnitt, kann aber grundsätzlich eine beliebige Innenquerschnittsform aufweisen. Die axialen Enden sind vorzugsweise offen und von ringförmigen Stirnflächen umgeben. Das Gehäuseelement kann seinerseits ebenfalls Anschlüsse aufweisen, um das erste Medium in das Gehäuseelement einzuleiten und/oder aus dem Gehäuseelement auszuleiten. Solche Anschlüsse können z.B. an den axialen Endbereichen des Gehäuseelementes vorgesehen sein.

Vorrichtungen dieser Art sind im Stand der Technik allgemein bekannt, z.B. als sogenannte Oxygenatoren, bei denen ein Stoffaustausch zwischen Blut als erstem Medium und einem Gas oder Gasgemisch als zweitem Medium erfolgt, insbesondere um Blut mit Sauerstoff anzureichern und von Kohlendioxid abzureichern. Die in diesem Fall eingesetzten Hohlfasern sind semipermeabel, nämlich nicht durchlässig für Blutbestandteile, aber stoffpermeabel für Gas, insbesondere für Sauerstoff und Kohlendioxid.

Solche Vorrichtungen sind auch bekannt in anderen Einsatzgebieten, z.B. als Vorrichtungen zur Durchführung einer Dialyse.

Die Erfindung bezieht sich auf Vorrichtungen der eingangs genannten Art, unabhängig von der konkreten Anwendung, bevorzugt aber auf Oxygenatoren.

Die Erfindung betrifft auch ein Herstellungsverfahren für solche Vorrichtungen. Auch die Herstellung ist im Stand der Technik grundsätzlich bekannt.

Die Herstellung erfolgt dadurch, dass in ein Gehäuseelement der zu bildenden Vorrichtung ein Hohlfaserpaket mit zwischen den axialen Enden des Gehäuseelementes erstreckten, insbesondere zumindest im Wesentlichen axial erstreckten, stoffpermeablen Hohlfasern angeordnet wird. Vorzugsweise ist ein solches Paket axial länger als das Gehäuseelement, so dass die axialen Enden des Hohlfaserpaketes aus dem Gehäuseelement herausragen, vorzugsweise beidseitig herausragen.

Ein solches Hohlfaserpaket kann gebildet werden durch Wickeln von zu Matten mit Kettfäden verketteten Hohlfasern, z.B. auf einem Wickelkern. Das Hohlfaserpaket kann aber auch durch Legen oder Falten von Matten erfolgen.

Das Paket kann, sofern es gewickelt ist, zusammen mit dem Wickelkern, der in diesem Fall von dem Hohlfaserpaket umgeben ist, vorzugsweise in dessen Zentrum liegt, in das Gehäuseelement eingesetzt werden. Insbesondere kann in einem solchen Fall ein Fluidfluss eines der Fluide, in der bevorzugten Anwendung z.B. von Blut, durch den Wickelkern vorgesehen sein, insbesondere wofür dieser - wie schon zuvor erwähnt - wenigstens einen Kanal aufweisen kann.

Nach dem Verschließen des Gehäuseelementes an wenigstens einem seiner axialen Enden, vorzugsweise an beiden Enden, mit einem bzw. jeweils einem Vergussdeckel werden die Hohlfasern an ihrem vom Vergussdeckel überdeckten axialen Endbereich zumindest untereinander, vorzugsweise auch mit der Gehäuseelementinnenwand, ggfs. auch mit einem Wickelkern mit einer Vergussmasse vergossen. Üblicherweise weist der Vergussdeckel dabei einen von der Deckelfläche axial erstreckten abstehenden Rohrabschnitt auf, der den axialen Endbereich der Hohlfasern umgibt, die aus dem Gehäuseelement herausragen. Der Vergussdeckel kann vorzugsweise hierdurch im Wesentlichen topfförmig ausgebildet sein. Deckelfläche und Rohrabschnitt können auch separate Elemente bilden, die zum Zweck des Vergießens verbunden werden.

Der auch als Verpottung bezeichnete Vergussvorgang wird üblicherweise in einer Zentrifuge vorgenommen, wobei bei dem Zentrifugieren die Vergussmasse in das mit dem Vergussdeckel verschlossene Gehäuseelement eingelassen wird, z.B. durch Anschlüsse im Vergussdeckel.

Gemäß dem bekannten Stand der Technik wird als Vergussmasse z.B. eine Polyurethanmasse eingesetzt, welche aushärtet und zu einer Verklebung der Hohlfasern untereinander und auch mit der Gehäuseelementinnenwand und dem vorzugsweise verwendeten Wickelkern führt. Eine solche Polyurethanmasse weist nach der Aushärtung faktisch keine Elastizität mehr auf, die eine reversible Verformung zulässt.

Nach dem Vergießen bzw. Verpotten und der Erhärtung oder Aushärtung der Vergussmasse wird der Vergussdeckel entfernt und die vergossenen Hohlfasern werden an ihren axialen durch die Vergussmasse vergossenen oder zuvor verschlossenen Enden eröffnet, z.B. durch Aufschneiden oder Aufsägen in einer Ebene senkrecht zur axialen Längserstreckungsrichtung der Hohlfasern. Anschließend wird das Gehäuseelement mit den darin befindlichen vergossenen und eröffneten Hohlfasern mit einem Deckelelement dicht verschlossen, wie zuvor beschrieben.

Vorzugsweise wird unter einem ausgehärteten Zustand ein solcher verstanden, in dem die Härtung der Vergussmasse abgeschlossen ist und nicht weiter fortschreitet. Ein erhärteter Zustand kann ein solcher sein, in dem eine Härtung der Vergussmasse gegenüber dem vorherigen Zustand beim Vergießen vorliegt, insbesondere der aber hinsichtlich der Härtung noch nicht abgeschlossen sein muss oder ist, insbesondere aber auch abgeschlossen sein kann.

Die vorbenannten Merkmale, insbesondere abgesehen von der Polyurethan-Vergussmasse, können vorzugsweise auch bei der Erfindung vorkommen.

Vor diesem geschilderten Hintergrund mit der Problematik der bisherigen Dichtungsart ist es eine Aufgabe der Erfindung eine Vorrichtung der eingangs genannten Art und ein Herstellungsverfahren der eingangs genannten Art bereitzustellen, welches eine einfache und dabei zuverlässige Erstellung einer Abdichtung zwischen der Stirnfläche des Gehäuseelements und dem Deckelelement und vorzugsweise auch zwischen Gehäuseelement und Hohlfaserpaket ermöglicht.

Diese Aufgabe wird erfindungsgemäß mit einem Herstellungsverfahren gelöst, bei dem aus der Vergussmasse selbst gleichzeitig mit dem Vergießen der Hohlfasern untereinander auch ein das Hohlfaserpaket radial außen umgebender, insbesondere in Umfangsrichtung um die Hohlfaserpaketlängsachse über volle 360 Grad umgebender äußerer Dichtungsring erstellt wird durch Hineinfließen der Vergussmasse in einen zwischen den Stirnflächen von Gehäuseelement und Vergussdeckel ausgebildeten Ringspalt. Vorzugsweise erstreckt sich dieser Ringspalt in Umfangsrichtung über volle 360 Grad in der Innenwandung des aus dem Gehäuseelement und dem Vergussdeckel gebildeten Gesamtgehäuse.

Der Ringspalt im Gesamtgehäuse ist somit in einer Richtung nach radial innen offen, so dass Vergussmasse aus dem Innenbereich des Gesamtgehäuses in diesen Ringspalt eindringen kann.

Vorzugsweise kann es vorgesehen sein nicht nur einen äußeren Dichtungsring aus der Vergussmasse zu erstellen, sondern auch einen inneren Dichtungsring. Dies ist insbesondere in solchen Ausführungen vorgesehen, in denen das Hohlfaserpaket als ein Wickel auf einem Wickelkern getragen ist, der im Gehäuse verbleibt. In diesem Fall gibt es nicht nur einen Ringspalt, in diesem Fall einen äußeren Ringspalt, sondern auch einen inneren Ringspalt. Bei solchen aufgewickelten Hohlfaserpaketen sind die Pakete vorzugsweise im Querschnitt senkrecht zur Längserstreckung ringförmig. Insbesondere gilt diese Querschnittsform dann auch für die Vergussmasse nach deren Erhärtung oder Aushärtung.

Bei Paketen ohne Wickelkern ist eine solche Ausführung mit innerem Dichtungsring vorzugsweise nicht vorgesehen. In einer solchen Ausführung ist das Hohlfaserpaket vorzugsweise im Querschnitt senkrecht zur Längserstreckung zylindrisch ausgebildet, weiter vorzugsweise wobei der Querschnitt vollständig von Hohlfasern gefüllt ist.

Im Verfahren kann vorgesehen sein, dass aus der Vergussmasse gleichzeitig mit dem Vergiessen der Hohlfasern untereinander am inneren Umfang des Hohlfaserpakets ein innerer Dichtungsring erstellt wird durch Hineinfließen der Vergussmasse in einen zwischen den Stirnflächen von einem Wickelkern, der das Hohlfaserpaket trägt, und einem Abdeckelement im Vergussdeckel ausgebildeten Ringspalt, insbesondere der sodann als innerer Ringspalt zu bezeichnen ist.

Der äußere und/oder innere Dichtungsring ist jeweils nach dem Vergießen und Erhärten oder Aushärten der Vergussmasse direkt aus dieser gebildet.

Gehäuseelement und Vergussdeckel grenzen vorzugsweise in allen möglichen Ausführungen an einer Trennebene aneinander an, wobei sie sich in der Trennebene kontaktieren. Gleiches kann bei vorgesehenem Wickelkern auch für diesen und einen Stopfen im Deckelelement gelten.

In einer möglichen Ausführung kann es vorgesehen sein, dass zumindest der äußere Ringspalt, vorzugsweise der äußere und der innere Ringspalt in axialer Richtung an die Trennebene angrenzt, insbesondere also nur in einem der beiden Elemente angeordnet ist und axial in Richtung zum anderen Element bis an die Trennebene heranreicht.

**In** einer anderen möglichen Ausführung kann zumindest der äußere Ringspalt, vorzugsweise auch der innere Ringspalt, in axialer Richtung um die Trennebene herumliegen, insbesondere ist der äußere Ringspalt dann im Gehäuseelement und im Vergussdeckel bzw. der innere Ringspalt im Wickelkern und Stopfen jeweils teilweise angeordnet.

Es kann so eine Vorrichtung der eingangs genannten Art hergestellt werden, bei der erfindungsgemäß an wenigstens einem der axialen Enden des Gehäuseelementes, vorzugsweise an beiden, aus der zwischen den Hohlfasern angeordneten Vergussmasse ein das Hohlfaserpaket außen umgebender äußerer Dichtungsring ausgebildet ist, der sich in radialer Richtung zumindest bereichsweise zwischen den axialen Stirnflächen des Gehäuseelementes und des Deckelelementes erstreckt und zwischen diesen komprimiert ist.

**In** der bevorzugten Weiterbildung kann an wenigstens einem der axialen Enden des Wickelkerns, der die Hohlfasern trägt, vorzugsweise an beiden, aus der zwischen den Hohlfasern angeordneten Vergussmasse auch ein am inneren Umfang des Hohlfaserpakets angeordneter innerer Dichtungsring ausgebildet sein, der sich in radialer Richtung zumindest bereichsweise zwischen den axialen Stirnflächen des Wickelkerns und eines Stopfens im Deckelelement erstreckt und zwischen diesen komprimiert ist.

Das Deckelelement kann so wie es auch schon zum Vergussdeckel beschrieben ist, vorzugsweise im Wesentlichen topfförmig ausgebildet sein, wofür das Deckelelement eine Deckelfläche und einen davon axial wegerstreckten Rohrabschnitt aufweist, die einstückig sind. Deckelfläche und Rohrabschnitt können auch separate Elemente bilden, die zum Zweck der Bildung des gesamten Vorrichtungsgehäuses verbunden werden. Der Rohrabschnitt kann dabei einen axialen Endbereich der vergossenen Hohlfasern umgeben. Die Deckelfläche liegt axial benachbart zu diesem Endbereich der Hohlfasern, insbesondere mit einem Abstand.

Die bereichsweise Erstreckung ist dabei vorzugsweise so zu verstehen, dass der äußere Dichtungsring in radialer Richtung betrachtet ausgehend vom radialen Innenrand der Stirnflächen sich nach radial außen erstreckt aber die jeweilige Stirnfläche nicht vollständig in radialer Richtung überdeckt. Vorzugsweise ist somit der äußere Dichtungsring in der fertigen Vorrichtung von außen nicht zugänglich, sondern zwischen den Stirnflächen beider Elemente eingeschlossen. Es kann aber auch eine Ausführung vorgesehen sein, bei welcher der Dichtungsring die Stirnflächen beider Elemente vollständig überdeckt oder sogar in radialer Richtung darüber hinausragt.

Unter einer Stirnfläche des Gehäuseelementes und/oder Deckelelementes und/oder Vergussdeckels und/oder Wickelkern und/oder Stopfens bzw. Abdeckelements wird vorzugsweise die jeweilige stirnseitige, insbesondere ringförmige Fläche am axialen Endbereich des betroffenen Elementes verstanden, die zumindest bereichsweise einen Dichtungszweck zwischen den sich gegenüberliegenden Elementen erfüllt. Solche dichtenden Stirnflächen liegen sich somit axial gegenüber, insbesondere kontaktieren sie sich zumindest bereichsweise direkt und bereichsweise indirekt über den jeweiligen Dichtungsring. Am axialen Ende eines Elementes können weiterhin andere Elemente / Flächenelemente angeordnet sein, z.B. zum Zweck der Befestigung, z.B. durch Verschraubung, Verrastung etc.

Vorzugsweise ist ein solcher erfindungsgemäßer äußerer Dichtungsring nach dem Vergießen durch solche Bereiche der Vergussmasse ausgebildet, welche radial außerhalb einer das Hohlfaserpaket umgebenden (gedachten) Hüllkurve liegen, insbesondere zumindest durch solche Bereiche ausgebildet, die radial außerhalb der Innenwandung des Gehäuseelementes und/oder Deckelelementes und/oder Vergussdeckels angeordnet sind.

Bei einem vorzugsweise vorgesehenen inneren Dichtungsring ist dieser nach dem Vergießen durch solche Bereiche der Vergussmasse ausgebildet, welche radial innerhalb einer an dem inneren Hohlfaserpaketumfang anliegenden (gedachten) Hüllkurve liegen, insbesondere zumindest durch solche Bereiche ausgebildet, die radial innen von der Außenwandung des Wickelkerns und/oder Stopfens und/oder Abdeckelements eines Vergussdeckels angeordnet sind.

Diese erfindungsgemäße Ausgestaltung hat bereits den Vorteil, dass die Vergussmasse vollständig raumfüllend in zumindest den äußeren Ringspalt, vorzugsweise auch in den inneren Ringspalt eindringt und so ein jeweiliger Ringbereich im Gehäuseelement oder Wickelkern, welcher den Dichtring aufnimmt, hohlraumfrei mit dem Dichtungsring gefüllt ist. Stagnationsbereiche können so bereits verhindert werden.

Vorzugsweise kann es vorgesehen sein, dass die Vergussmasse durch ein flüssig oder pastös, insbesondere zumindest fließfähiges, applizierbares erhärtendes Elastomer ausgebildet ist mit einer Härte, insbesondere im erhärteten oder ausgehärteten Zustand, kleiner Shore A 100, bevorzugt kleiner Shore A 60, weiter bevorzugt kleiner Shore A 30.

Hierdurch wird eine reversible Elastizität des gebildeten äußeren und/oder inneren Dichtungsringes ermöglicht, wie sie von üblichen Dichtringen bekannt ist. So kann die Vergussmasse neben dem Verguss der Hohlfasern untereinander auch besonders gut die für eine Dichtung nötigen Eigenschaften bereitstellen, insbesondere im Gegensatz zu üblichem Polyurethan.

Besonders bevorzugt ist es vorgesehen, als Vergussmasse ein Silikon / Silikonkautschuk einzusetzen, insbesondere das flüssig in einer Zentrifuge appliziert werden kann und dann erhärtet/aushärtet z.B. durch Vernetzen. Ebenso ist es möglich als Vergussmasse Latex einzusetzen.

Der mit der Vergussmasse zu füllende äußere Ringspalt wird vorzugsweise definiert durch eine axial und radial nach innen offene Ausnehmung in der Stirnfläche vom Gehäuseelement und/oder vom Vergussdeckel. Es kann eine Ausnehmung also nur in einem der beiden Elemente stirnseitig vorgesehen sein oder auch in beiden. Der zu füllende vorzugsweise vorgesehene innere Ringspalt wird beispielsweise definiert durch eine axial und radial nach außen offene Ausnehmung in der Stirnfläche vom Wickelkern und/oder vom Abdeckelement im Vergussdeckel. Es kann so auch beim inneren Ringspalt eine Ausnehmung also nur in einem der beiden Elemente stirnseitig vorgesehen sein oder auch in beiden.

Je nach Formgestaltung des Ringspaltes zwischen Gehäuseelement und Vergussdeckel oder Wickelkern und Abdeckelement können sich unterschiedliche Geometrien des jeweiligen Dichtungsringes und damit verschiedene realisierbare Dichtungsmöglichkeiten ergeben.

Zumindest zum Abschluß des Vergießens und Erhärtens oder Aushärtens der Vergussmasse ist der gebildete äußere und/oder innere Dichtungsring einstückig bzw. stoffschlüssig verbunden mit der Vergussmasse in den Bereichen zwischen den axialen Enden der vergossenen Hohlfasern. Vorzugsweise bleibt er dies auch in der fertig hergestellten Vorrichtung zum Stoffaustausch, dies ist aber nicht zwingend nötig.

So kann das Verfahren in einer möglichen Ausführung vorsehen, dass mit dem Eröffnen der Hohlfaserenden, insbesondere durch Abtrennen von verklebten Hohlfaserenden oder auch durch Abtrennen von herstellerseitig geschlossenen Hohlfaserenden auch der äußere und/oder innere Dichtungsring vom verklebten Hohlfaserpaket getrennt wird.

Dies kann z.B. erfolgen, wenn der Dichtungsring in einer Konfiguration erstellt ist, in welcher dieser nach dem Abnehmen des Vergussdeckels und/oder Abdeckelementes einen von der Ringspaltöffnung axial in Richtung zum Gehäuseelement zurückgerichteten Bereich aufweist an seinem radial außenliegenden Ende und/oder axial in Richtung zum Wickelkern zurückgerichteten Bereich aufweist an seinem radial innenliegenden Ende und mit diesem Bereich in einer zumindest in axialer Richtung offenen Ausnehmung, z.B. Ringnut, in der Stirnfläche des Gehäuseelementes und/oder Wickelkerns einliegt. Diese Ringnut im Gehäuseelement oder Wickelkern stellt zum Zeitpunkt des Vergießens einen hinterschnittenen Bereich des jeweiligen Ringspaltes dar.

Das Abtrennen der geschlossenen Hohlfaserenden kann z.B. bündig zur Stirnfläche des Gehäuseelementes und/oder Wickelkerns erfolgten, wodurch der gebildete äußere und/oder innere Dichtungsring in der Ausnehmung / Ringnut hohlraumfüllend verbleibt und vom Hohlfaserpaket getrennt wird.

Durch Aufsetzen des Deckelelementes und/oder Stopfens kann der so gebildete äußere und/oder innere Dichtungsring zwischen den Stirnflächen von Deckelelement und Gehäuseelement bzw. Stopfen und Wickelkern komprimiert werden, z.B. mittels eines vom Deckelelement zum Gehäuseelement oder vom Stopfen zum Wickelkern weisenden ringförmigen Vorsprungs, der gegenüberliegend zur Ausnehmung / Ringnut positioniert ist. Insbesondere kann der Stopfen, vorzugsweise wenn er separat zum Deckelelement ist, am Wickelkern befestigt sein, z.B. durch Verrasten oder Verschrauben oder dergleichen.

Eine demgegenüber bevorzugte Ausführung sieht hingegen vor, dass nach dem Eröffnen der Hohlfaserenden, insbesondere durch Abschneiden von vergossenen Hohlfaserenden oder auch durch Abtrennen von herstellerseitig geschlossenen Hohlfaserenden, der äußeren und/oder innere Dichtungsring stoffschlüssig / einstückig am verklebten Hohlfaserpaket befestigt bleibt und durch Aufsetzen des Deckelelementes zwischen den Stirnflächen von Deckelelement und Gehäuseelement komprimiert wird und/oder durch Aufsetzen des Stopfens zwischen den Stirnflächen von Stopfen und Wickelkern komprimiert wird.

Insbesondere ist es hierfür vorgesehen die geschlossenen Hohlfaserenden in einer Ebene abzutrennen, die beabstandet ist, insbesondere mindestens um die gewünschte Dicke des Dichtungsringes beabstandet ist, zur distalen Stirnflächenebene, insbesondere also zur am weitesten axial außenliegenden Stirnflächenebene des Gehäuseelementes und/oder Wickelkerns.

Vorzugsweise ist die Trennebene beabstandet von der distalen Stirnflächenebene des Gehäuseelementes und/oder Wickelkerns um ein Maß das mindestens 20% größer ist als die gewünschte axiale Dicke des gebildeten Dichtungsringes, weiter bevorzugt mindestens 40% größer ist als die gewünschte axiale Dicke des gebildeten Dichtungsringes, insbesondere an seinem radial innen liegenden Bereich.

Diese Ausführung gestattet es, dass der äußere und/oder innere Dichtungsring auch bei der fertig hergestellten Vorrichtung für den Stoffaustausch mit der Vergussmasse zwischen den Hohlfasern einstückig ist / stoffschlüssig verbunden ist. Durch diese stoffschlüssig bestehenbleibende Verbindung zum vergossenen Hohlfaserpaket wird der Vorteil erschlossen, dass mit der Abdichtung des Deckelelementes gegenüber dem Gehäuseelement durch den äußeren Dichtungsring auch gleichzeitig das Hohlfaserpaket radial aussen gegenüber dem Gehäuseelement und dem Deckelelement gedichtet ist. Weiterhin wird bei vorgesehenem inneren Dichtungsring mit dem Aufsetzen des Stopfens auf den Wickelkern auch gleichzeitig das Hohlfaserpaket radial innen gegenüber dem Deckelelement gedichtet.

Es kann durch die Formgebung des zwischen Vergussdeckel und Gehäuseelement und/oder zwischen Abdeckelement im Vergussdeckel und Wickelkern definierten Ringspaltes z.B. ein äußerer Dichtungsring ausgebildet werden, der nach Entfernen des Vergussdeckels in einer radial innen in der Stirnfläche des Gehäuseelementes angeordneten Ausnehmung einliegt und/oder ein innerer Dichtungsring ausgebildet werden, der nach Entfernen des Vergussdeckels und Abdeckelements in einer radial außen in der Stirnfläche des Wickelkerns angeordneten Ausnehmung einliegt

Dabei kann der äußere Dichtungsring z.B. bündig liegen mit der distalen Stirnflächenebene des Gehäuseelements, vorzugsweise aber auch in axialer Richtung über den die Ausnehmung umgebenden Stirnflächenbereich, insbesondere die distale Stirnflächenebene des Gehäuseelementes überstehen. Der innere Dichtungsring kann z.B. bündig liegen mit der distalen Stirnflächenebene des Wickelkerns, vorzugsweise aber auch in axialer Richtung über den radial innen von der Ausnehmung liegenden Stirnflächenbereich, insbesondere die distale Stirnflächenebene des Wickelkerns überstehen. So kann besonders einfach eine Komprimierung des Dichtungsringes durch Aufsetzen des Deckelelementes und/oder des Stopfens erzielt werden, insbesondere wobei die Komprimierung in die jeweilige Ausnehmung hinein erfolgt.

Bzgl. des äußeren Dichtungsringes kann das Deckelelement dabei die Komprimierung z.B. mit einer insgesamt nur in einer Ebene ausgebildeten, insbesondere stufenlosen, axialen Stirnfläche vornehmen oder auch durch einen an der Stirnfläche des Deckelelementes angeordneten, vorzugsweise radial innen angeordneten, axialen, vorzugsweise ringförmigen Vorsprung, der bereichsweise in die Ausnehmung eingreift. Bzgl. des inneren Dichtungsringes kann der Stopfen dabei die Komprimierung z.B. mit einer insgesamt nur in einer Ebene ausgebildeten, insbesondere stufenlosen, axialen Stirnfläche vornehmen oder auch durch einen an der Stirnfläche des Stopfens angeordneten, vorzugsweise radial außen angeordneten, axialen, vorzugsweise ringförmigen Vorsprung, der bereichsweise in die Ausnehmung eingreift.

Ebenso kann der gebildete äußere Dichtungsring auf einem in nur einer einzigen Stirnflächenebene, insbesondere auf einem in der radialen Erstreckung ohne Ausnehmung ausgebildeten, planen Oberflächenbereich der Stirnfläche des Gehäuseelementes in einer vorbestimmten Höhe aufliegen und durch Aufsetzen des Deckelelementes in einer axial und radial nach innen offenen Ausnehmung in der Stirnfläche des Deckelelementes mit einer Tiefe kleiner als die vorbestimmte Höhe komprimiert werden.

Ein vorzugsweise gebildeter innerer Dichtungsring kann auf einem in nur einer einzigen Stirnflächenebene, insbesondere auf einem in der radialen Erstreckung ohne Ausnehmung ausgebildeten, planen Oberflächenbereich der Stirnfläche des Wickelkerns in einer vorbestimmten Höhe aufliegen und durch Aufsetzen des Stopfens in einer axial und radial nach außen offenen Ausnehmung in der Stirnfläche des Stopfens mit einer Tiefe kleiner als die vorbestimmte Höhe komprimiert werden.

Allgemein kann bzgl. des äußeren Dichtungsringes bei der Vorrichtung an einer der axialen Stirnflächen von Gehäuseelement oder Deckelelement radial innen ein axialer, vorzugsweise ringförmiger, Vorsprung angeordnet sein, der zumindest bereichsweise in die axial offene Ausnehmung in der Stirnfläche des gegenüberliegenden Elementes einliegt. Weiterhin kann allgemein bzgl. des inneren Dichtungsringes bei der Vorrichtung an einer der axialen Stirnflächen von Wickelkern oder Stopfen radial außen ein axialer, vorzugsweise ringförmiger, Vorsprung angeordnet sein, der zumindest bereichsweise in die axial offene Ausnehmung in der Stirnfläche des gegenüberliegenden Elementes einliegt.

Ebenso kann die Stirnfläche des Deckelelementes oder des Stopfens mit einer Ausnehmung den überstehenden Dichtungsring übergreifen, vorzugsweise, wobei diese Ausnehmung eine Tiefe hat die kleiner ist als der Überstand, um die Komprimierung zu gewährleisten.

**In** der fertigen Vorrichtung kann bzgl. des äußeren Dichtungsringes allgemein somit vorgesehen sein, dass die axiale Stirnfläche vom Gehäuseelement und/oder vom Deckelelement radial innen eine axial und radial nach innen offene Ausnehmung aufweist, in welcher der äußere Dichtring komprimiert einliegt. **In** der fertigen Vorrichtung kann bzgl. des fakultativ vorgesehenen inneren Dichtungsringes allgemein vorgesehen sein, dass die axiale Stirnfläche vom Wickelkern und/oder vom Stopfen radial außen eine axial und radial nach außen offene Ausnehmung aufweist, in welcher der innere Dichtring komprimiert einliegt.

Soweit in den beschriebenen Ausführungen Ausnehmungen und/oder Vorsprünge an den Stirnflächen von Elementen, z.B. von Deckelelement und/oder Gehäuseelement und/oder Vergussdeckel und/oder Abdeckelement und/oder Stopfen erläutert werden ist vorzugsweise zu verstehen, dass diese sich in Umfangsrichtung um die Hohlfaserpaketlängsachse über volle 360 Grad erstrecken, insbesondere also ringförmig ausgebildet sind.

Eine weiterhin mögliche Ausführung kann es vorsehen, dass der äußere Dichtungsring in radialer Richtung nach außen über einen Vorsprung, vorzugsweise radial innen liegenden Vorsprung an einer der Stirnflächen von Gehäuseelement (bevorzugt) oder Deckelelement hinweg und darüber hinauskragend erstreckt ist und durch einen gestuften Bereich an der Ausnehmung des gegenüberliegenden Elementes axial abgeknickt ist in einen Bereich hinein, der radial außen vom Vorsprung angeordnet ist, vorzugsweise der auch axial hinter den Vorsprung zurücktretend angeordnet ist.

Eine mögliche analoge Ausführung kann es vorsehen, dass der vorzugsweise vorhandene innere Dichtungsring in radialer Richtung nach innen über einen Vorsprung, vorzugsweise radial außen liegenden Vorsprung an einer der Stirnflächen von Wickelkern (bevorzugt) oder Stopfen hinweg und darüber hinauskragend erstreckt ist und durch einen gestuften Bereich an der Ausnehmung des gegenüberliegenden Elementes axial abgeknickt ist in einen Bereich hinein, der radial innen vom Vorsprung angeordnet ist, vorzugsweise der auch axial hinter den Vorsprung zurücktretend angeordnet ist.

Im Verfahren kann der äußere Dichtungsring z.B. so gebildet werden, dass dieser einen radial innenliegenden axialen Vorsprung in der Stirnfläche des Gehäuseelementes überdeckt und in radialer Richtung nach außen überkragt und beim Aufsetzen des Deckelelementes durch eine den Vorsprung umgreifende Ausnehmung in der Stirnfläche des Deckelelementes komprimiert und in axialer Richtung abgeknickt wird.

Analog kann der fakultativ vorhandene innere Dichtungsring z.B. so gebildet werden, dass dieser einen radial außenliegenden axialen Vorsprung in der Stirnfläche des Wickelkerns überdeckt und in radialer Richtung nach innen überkragt und beim Aufsetzen des Stopfens durch eine den Vorsprung umgreifende Ausnehmung in der Stirnfläche des Stopfens komprimiert und in axialer Richtung abgeknickt wird.

Bevorzugte Ausführungsformen werden anhand der Figuren erläutert.

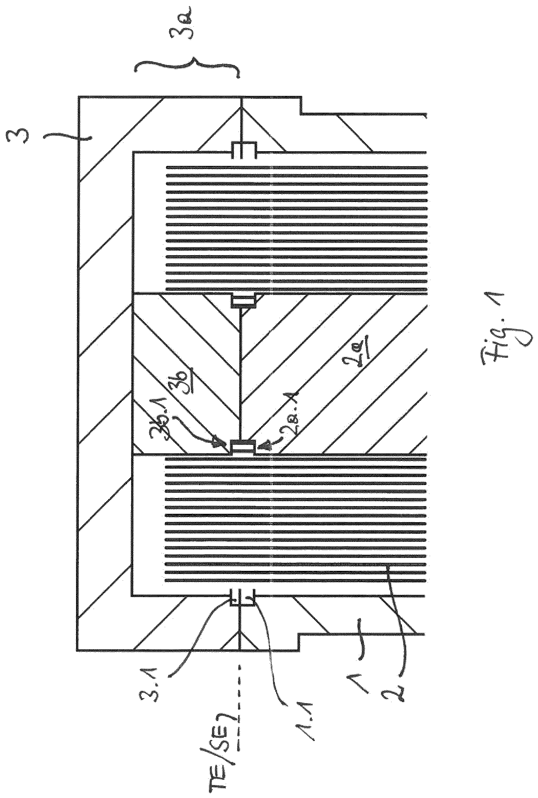

Figur 1 zeigt einen ersten Schritt des Verfahrens zur Herstellung, bei dem in das Gehäuseelement 1 ein Hohlfaserpaket 2 eingelegt wird, welches axial über die Trennebene TE zwischen Gehäuseelement 1 und Vergussdeckel 3 bzw. die distale Stirnflächenebene SE des Gehäuseelementes 1 übersteht. Mit dem Vergussdeckel 3 wird das Gehäuseelement 1 axial geschlossen und das Hohlfaserpaket 2 endseitig mit einem rohrförmigen Abschnitt 3a des Vergussdeckels 3 umgriffen.

Das Hohlfaserpaket ist hier z.B. gebildet durch ein Wickel von Hohlfasern, die z.B. zu wenigstens einer Matte mit Kettfäden verbunden sind, wobei das Wickel auf einem Wickelkern 2a getragen wird, insbesondere der auch später im Gehäuseelement 1 verbleiben kann. Am Vergussdeckel 3 ist ein Abdeckelement 3b angeordnet, welches dem Wickelkern 2a axial gegenüberliegt, insbesondere dessen axiale Stirnfläche berührend überdeckt. Das Abdeckelement 3b kann mit dem Vergussdeckel 3 einstückig ausgebildet sein oder ist separat zu diesem. Z.B. kann es bei separater Ausführung zwischen dem Vergussdeckel 3 und dem Wickelkern 2a eingeklemmt sein.

Beide ringförmigen Stirnflächen von Gehäuseelement 1 und Vergussdeckel 3 weisen radial innenliegend jeweilige Ausnehmungen 1.1 und 3.1 auf, die axial und nach radial innen offen sind und so einen Ringspalt auf der Innenwandung der geschlossenen Anordnung, also des Gesamtgehäuses bilden. In dieser Ausführung liegt der Ringspalt 1.1, 3.1 axial um die Trennebene TE beider Elemente 1, 3 herum, die mit der distalen Stirnflächenebene SE des Gehäuseelementes 1 zusammenfällt. Die distale Stirnfläche des Gehäuseelementes liegt vorzugsweise in derselben Ebene wie die distale Stirnfläche des Wickelkerns 2a.

Zusätzlich zu dem so gebildeten äußeren Ringspalt, der sich radial außen um die Hohlfasern herum ergibt und bei der Erfindung zumindest vorgesehen ist, sieht diese Ausführung weiterhin vor, dass sich auch radial innen zum Hohlfaserpaket 2 ein innerer Ringspalt ergibt. Dieser innere Ringspalt ist zwischen dem Wickelkern 2a und dem Abdeckelement 3b ausgebildet. Hierfür weisen das Abdeckelement 3b und der Wickelkern 2a jeweils radial außenliegend jeweilige Ausnehmungen 3b.1 und 2a.1 auf, die axial und nach radial außen offen sind. Auch der innere Ringspalt 2a.1, 3b.1 liegt axial um die Trennebene TE herum.

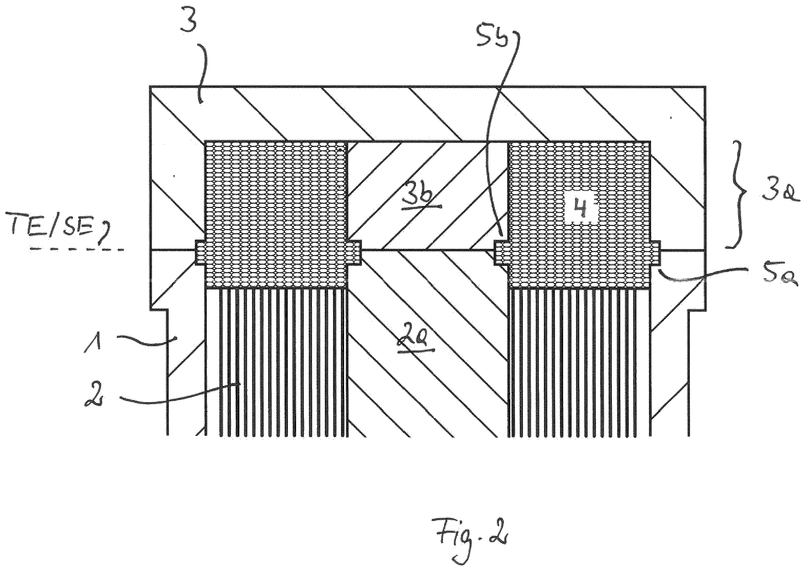

Figur 2 zeigt dieselbe Anordnung nach dem axial endseitigen Verguss. Die Vergussmasse 4 hat hierbei die Hohlfaserenden überdeckt, evtl. verschlossen, sofern diese nicht herstellerseitig geschlossen waren, und ist auch in den durch die Ausnehmungen 1.1 und 3.1 gebildeten äußeren Ringspalt, sowie in den durch die Ausnehmungen 3b.1 und 2a.1. gebildeten inneren Ringspalt eingedrungen und hat so einen äußeren Dichtungsring 5a gebildet, der das Hohlfaserpaket 2 außen umgibt und einen inneren Dichtring 5b gebildet, der innen entlang des Innenumfangs des Hohlfaserpakets 2 angeordnet ist.

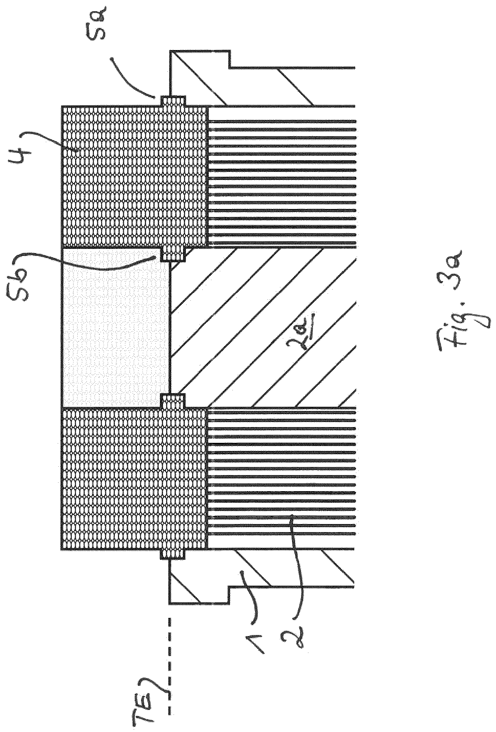

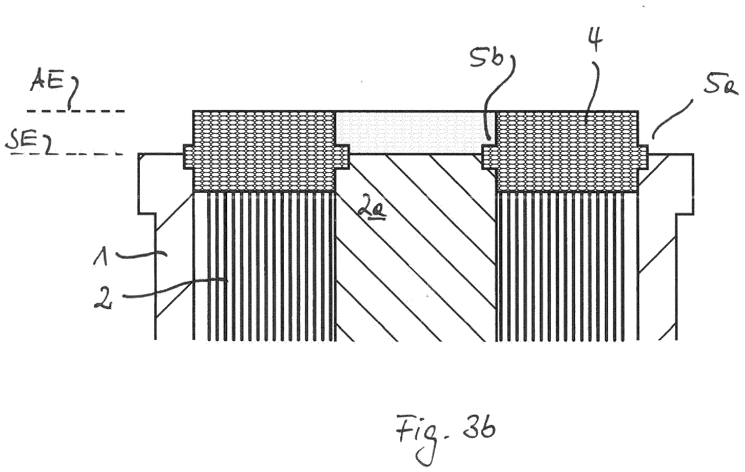

Figur 3a zeigt die Anordnung nach Abnahme des Vergussdeckels 3 und auch des Abdeckelementes 3b. Figur 3b zeigt dieselbe Anordnung nach Abtrennung eines Teils der vergossenen Hohlfasern um diese zu öffnen. Die Abtrennung erfolgt in der Abtrennebene AE in einem Abstand zu den Dichtungsringen 5a/5b, der in dieser Ausführung über die distale Stirnflächenebene SE des Gehäuseelementes 1 übersteht.

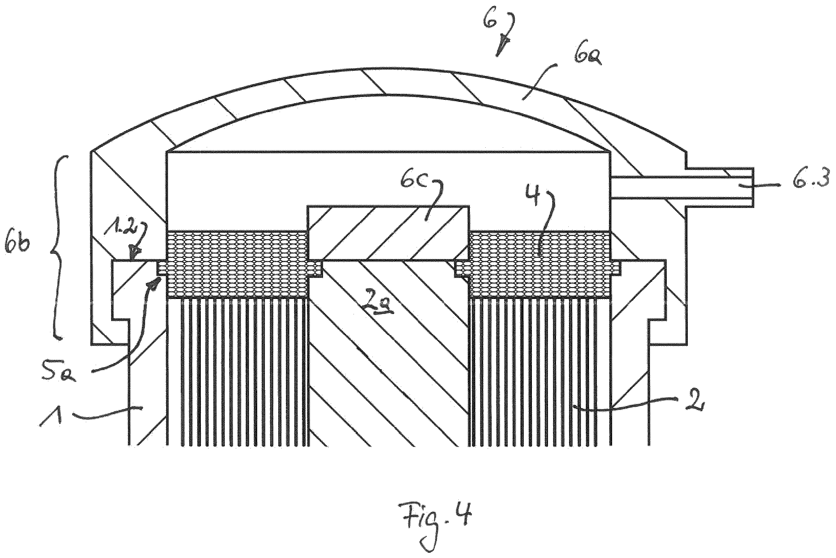

Figur 4 zeigt die Situation nach Aufsetzen des Deckelelementes 6 der Vorrichtung. Das Deckelelement 6 ist, insbesondere ähnlich wie der Vergussdeckel 3, topfförmig ausgebildet und weist eine Deckelfläche, vorzugsweise nach außen gewölbte Deckelfläche 6a auf, von der sich axial in Richtung zum Gehäuseelement 1 ein rohrförmiger Abschnitt 6b erstreckt, der die vergossenen Hohlfaserenden bereichsweise umgibt.

Das Deckelelement 6 hat weiterhin einen Anschluß 6.3 um Gas zu den offenen Hohlfasern zu oder von diesen abzuführen. Hier hat das Deckelelement 6 in dem Stirnflächenbereich, der mit der distalen Stirnfläche 1.2 des Gehäuseelementes 1 zum Zweck der Dichtung zusammenwirkt, keine Ausnehmung und komprimiert den äußeren Dichtungsring 5a durch Auflegen der Stirnfläche auf den axial überstehenden Bereich des äußeren Dichtungsringes 5a.

Der innere Dichtungsring 5b wird von einem Stopfen 6c komprimiert, insbesondere der einstückiger Teil des Deckelelementes 6 sein kann oder auch separat zu diesem ausgeführt sein kann. Der Stopfen 6c liegt kontaktierend auf dem Wickelkern 2a auf. Insbesondere kann der Stopfen 6c, vorzugsweise wenn er separat zum Deckelelement 6 ist, am Wickelkern 2a befestigt sein, z.B. durch Verrasten oder Verschrauben oder dergleichen. Dies kann auch für alle möglichen, ggfs. auch nicht gezeigten Ausführungen gelten.

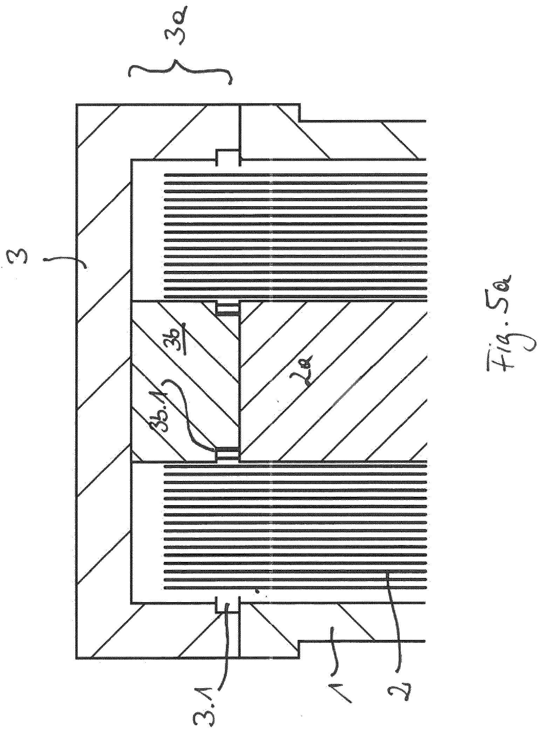

Figur 5a zeigt eine Alternative, bei welcher die Stirnfläche des Gehäuseelementes 1 in einer einzigen Ebene liegt, also keine Ausnehmung aufweist. **In** derselben Ebene liegt die ebenso stufenlos plane Stirnfläche des Wickelkerns 2a und weist ebenso keine Ausnehmung auf. Eine axial und radial nach innen offene Ausnehmung 3.1 ist nur in der Stirnfläche des Vergussdeckels 3 zur Bildung der äußeren Ringnut und eine axial und radial nach außen offene Ausnehmung 3b.1 nur in dem Abdeckelement 3b zur Bildung der inneren Ringnut angeordnet.

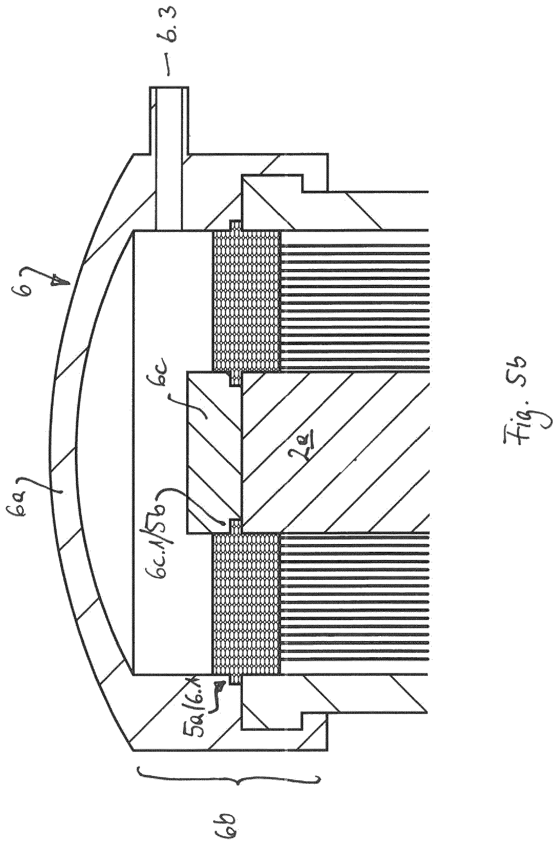

**In** der fertigen Vorrichtung gemäß der Figur 5b liegt der äußere Dichtungsring 5a nur in einer radial nach innen und axial offenen Ausnehmung 6.1 ein und wird durch diese komprimiert, da die Tiefe der Ausnehmung 6.1 geringer ist als die Höhe des Dichtungsringes 5a über der Stirnfläche des Gehäuseelementes 1. Der innere Dichtungsring 5b liegt nur in einer radial nach außen und axial offenen Ausnehmung 6c.1 ein und wird durch diese komprimiert, da die Tiefe der Ausnehmung 6c.1 im Stopfen 6c geringer ist als die Höhe des Dichtungsringes 5b über der Stirnfläche des Wickelkerns 2a.

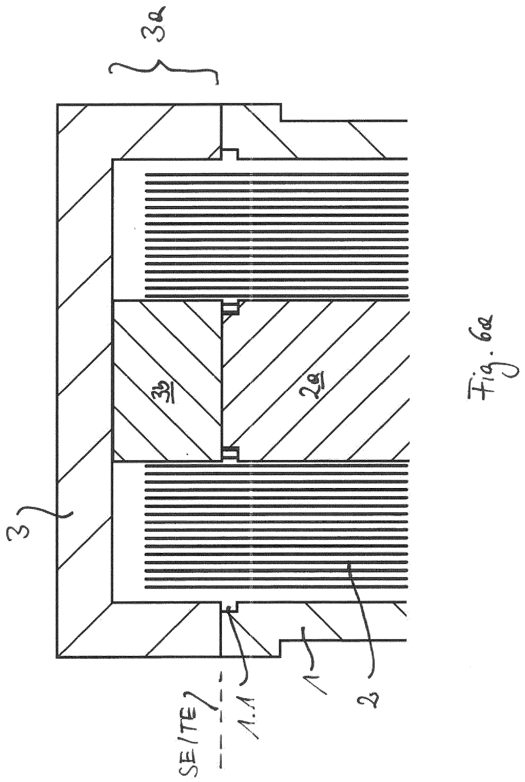

Figur 6a zeigt eine Ausführung, bei der nur eine radial nach innen und axial offene Ausnehmung 1.1 im Gehäuseelement 1 angeordnet ist. Die axiale Stirnfläche des Vergussdeckels 3 liegt in einer einzigen Ebene (ohne Ausnehmung) und überdeckt die Ausnehmung 1.1 in der durch die Ausnehmung 1.1 gestuften Stirnfläche des Gehäuseelementes 1.

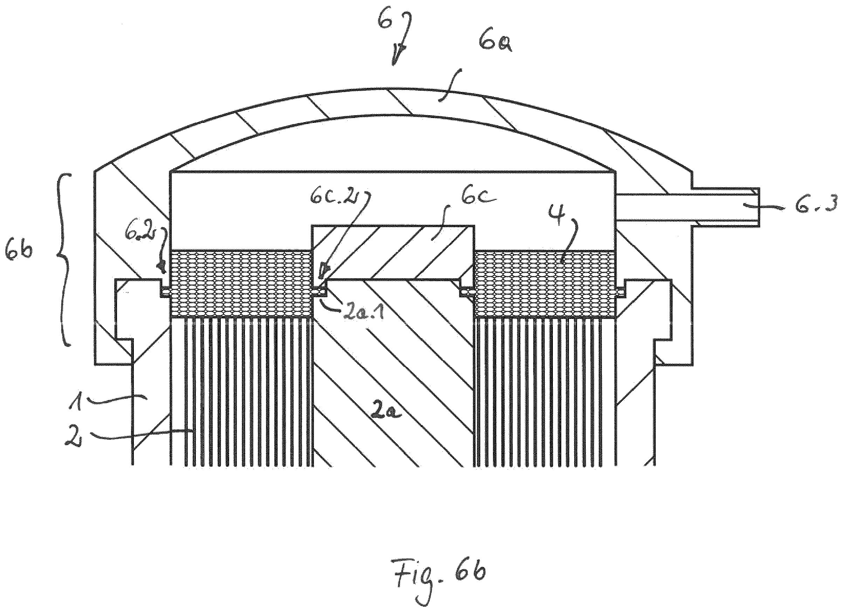

Der gemäß Figur 6b gebildete äußere Dichtungsring 5a liegt damit in der distalen Stirnflächenebene SE des Gehäuseelementes 1 und ragt nicht über die Ausnehmung 1.1 hinaus. Zur Kompression weist nun das Deckelelement 6 an seiner Stirnfläche radial innen einen zum Gehäuseelement 1 gerichteten axialen ringförmigen Vorsprung 6.2 auf, der teilweise in die mit dem Dichtungsring 5a gefüllte Ausnehmung 1.1 an der Stirnfläche des Gehäuseelementes 1 eingreift.

Bezogen auf den inneren Dichtungsring 5b gilt, dass dieser ebenso in der distalen Stirnflächenebene SE des Gehäuseelementes 1 bzw. des Wickelkerns 2b liegt und nicht über die Ausnehmung 2b.1 hinausragt. Zur Kompression weist nun der Stopfen 6c an seiner Stirnfläche radial außen einen zum Wickelkern 2a gerichteten axialen Ringvorsprung 6c.2 auf, der teilweise in die mit dem Dichtungsring 5b gefüllte Ausnehmung 2a.1 an der Stirnfläche des Wickelkerns 2a eingreift.

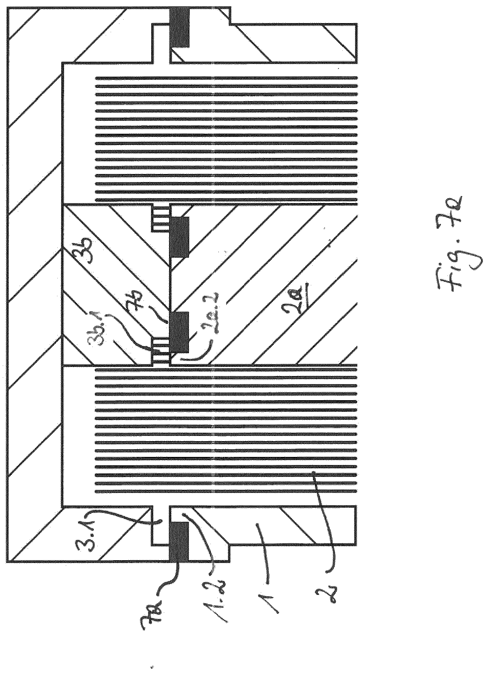

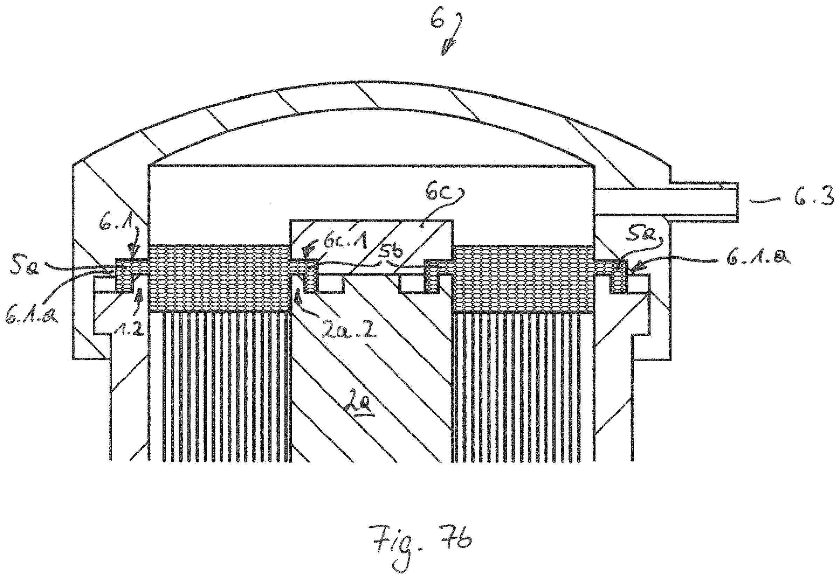

Die Figuren 7a und 7b zeigen eine Ausführung, bei welcher der fertig gebildete äußere Dichtungsring 5a in der fertigen Vorrichtung (Figur 7b) einen radial innen liegenden Vorsprung 1.2 im Gehäuseelement 1 überdeckt und radial auch darüber in eine Richtung nach radial außen hinauskragt. Der hinauskragende Teil des Dichtungsringes 5a ist durch eine Stufe 6.1.a der axial und radial nach innen offenen Ausnehmung 6.1 in der Stirnfläche des Deckelelementes 6 in axialer Richtung abgeknickt und komprimiert. Die Stufe 6.1.a begrenzt die Ausnehmung 6.1 radial außen.

Für den inneren Dichtungsring 5b gilt, dass dieser in der fertigen Vorrichtung (Figur 7b) einen radial außen liegenden Vorsprung 2a.2 im Wickelkern 2a überdeckt und radial auch darüber in eine Richtung nach radial innen hinauskragt. Der hinauskragende Teil des Dichtungsringes 5b ist durch eine Stufe der axial und radial nach außen offenen Ausnehmung 6c.1 in der Stirnfläche des Stopfens 6c in axialer Richtung abgeknickt und komprimiert. Die Stufe begrenzt die Ausnehmung 6c.1 radial innen.

In Figur 7a ist erkennbar, dass ein den Vorsprung 1.2. überkragender äußerer Dichtungsrichtung 5a hergestellt werden kann beim Vergießen durch Auffüllen des radial außen ausgenommenen Bereiches radial hinter dem Vorsprung 1.2, z.B. mit einem Füllring 7a, der vor Aufsetzen des Deckelelementes 6 wieder entfernt wird. Es ergibt sich durch den Füllring 7a ein radial außenliegender in axialer Richtung unterstützter Bereich des Ringspaltes 3.1, so dass ein radial geradlinig erstreckter äußerer Dichtungsring 5a hergestellt werden kann, der den Vorsprung 1.2 in radialer Richtung nach außen überragt. In den beim Vergießen vom Füllring 7a gefüllten Bereich kann nach Entfernen des Füllringes 7a der äußere Dichtungsring 5a durch das Deckelelement 6 abgeknickt werden.

Weiterhin kann ein den Vorsprung 2a.2 überkragender innerer Dichtungsring 5b hergestellt werden, beim Vergießen durch Auffüllen des radial innen ausgenommenen Bereiches radial innen hinter dem Vorsprung 2a.2, z.B. mit einem Füllring 7b, der vor Aufsetzen des Stopfens 6c wieder entfernt wird. Es ergibt sich durch den Füllring 7b ein radial innenliegender in axialer Richtung unterstützter Bereich des Ringspaltes 3b.1, so dass ein radial geradlinig erstreckter innerer Dichtungsring 5b hergestellt werden kann, der den Vorsprung 2a.2 in radialer Richtung nach innen überragt. In den beim Vergießen vom Füllring 7b gefüllten Bereich kann nach Entfernen des Füllringes 7b der innere Dichtungsring 5b durch den Stopfen 6c abgeknickt werden.

## Patentansprüche

1. Vorrichtung für den Stoffaustausch zwischen einem ersten Medium, insbesondere Blut, und einem zweiten Medium, insbesondere einem Gas/Gasgemisch, umfassend ein Gehäuseelement (1) in dem ein Hohlfaserpaket (2) mit zwischen den axialen Enden des Gehäuseelementes (1) axial erstreckten stoffpermeablen und auf einem Wickelkern aufgewickelten Hohlfasern angeordnet ist, die an ihrem jeweiligen axialen Endbereich zumindest untereinander mit einer Vergussmasse (4) vergossen sind und die von dem ersten Medium umströmbar und vom zweiten Medium durchströmbar sind, wobei das Gehäuseelement (1) an wenigstens einem der axialen Enden, vorzugsweise an beiden axialen Enden, mit einem Deckelelement (6) verschlossen ist, insbesondere welches einen Medienanschluß (6.1) aufweist, der mit den axial offenen Hohlfaserenden in Fluidverbindung steht, **dadurch gekennzeichnet, dass** an wenigstens einem der axialen Enden des Gehäuseelementes (1), vorzugsweise an beiden, aus der zwischen den Hohlfasern angeordneten Vergussmasse (4) ein das Hohlfaserpaket (2) umgebender äußerer Dichtungsring (5a) ausgebildet ist, der sich in radialer Richtung zumindest bereichsweise zwischen den axialen Stirnflächen des Gehäuseelementes (1) und des Deckelelementes (6) erstreckt und zwischen diesen komprimiert ist und an wenigstens einem der axialen Enden des Wickelkerns (2a), der die Hohlfasern (2) trägt vorzugsweise an beiden, aus der zwischen den Hohlfasern angeordneten Vergussmasse (4) ein am inneren Umfang des Hohlfaserpakets (2) angeordneter innerer Dichtungsring (5b) ausgebildet ist, der sich in radialer Richtung zumindest bereichsweise zwischen den axialen Stirnflächen des Wickelkerns (2a) und eines Stopfens (6c) im Deckelelement (6) erstreckt und zwischen diesen komprimiert ist.

2. Vorrichtung nach Anspruch 1, **dadurch gekennzeichnet, dass** der äußere und/oder innere Dichtungsring (5a, 5b) mit der Vergussmasse (4) zwischen den Hohlfasern einstückig ist / stoffschlüssig verbunden ist.

3. Vorrichtung nach einem der vorherigen Ansprüche, **dadurch gekennzeichnet, dass** die Vergussmasse (4) durch ein Elastomer ausgebildet ist, vorzugsweise mit einer Härte kleiner Shore A 100, bevorzugt kleiner Shore A 60, weiter bevorzugt kleiner Shore A 30, insbesondere die Vergussmasse (4) aus einem Silikon / Silikonkautschuk ausgebildet ist.

4. Vorrichtung nach einem der vorherigen Ansprüche, **dadurch gekennzeichnet, dass** die axiale Stirnfläche vom Gehäuseelement (1) und/oder vom Deckelelement (6) radial innen eine axial und radial nach innen offene Ausnehmung (1.1, 6.1) aufweist, in welcher der äußere Dichtungsring (5a) komprimiert einliegt und/oder die axiale Stirnfläche vom Wickelkern (2a) und/oder vom Stopfen (6c) im Deckelelement (6) radial außen eine axial und radial nach außen offene Ausnehmung (1.1, 6.1) aufweist, in welcher der innere Dichtungsring (5b) komprimiert einliegt.

5. Vorrichtung nach Anspruch 4, **dadurch gekennzeichnet, dass** an einer der Stirnflächen von Gehäuseelement (1) oder Deckelelement (6) radial innen ein axialer, vorzugsweise ringförmiger, Vorsprung (6.2, 1.2) angeordnet ist, der zumindest bereichsweise in die axial offene Ausnehmung (1.1, 6.1) in der Stirnfläche des gegenüberliegenden Elementes (1, 6) einliegt oder an einer der Stirnflächen von Wickelkern (2a) oder dem Stopfen (6c) im Deckelelement (6) radial außen ein axialer, vorzugsweise ringförmiger, Vorsprung (6c.2, 2a.2) angeordnet ist, der zumindest bereichsweise in die axial offene Ausnehmung (2a.1, 6c.1) in der Stirnfläche des gegenüberliegenden Elementes (6c, 2a) einliegt.

6. Vorrichtung nach Anspruch 5, **dadurch gekennzeichnet, dass** der äußere Dichtungsring (5a) in radialer Richtung nach außen über den Vorsprung (1.2) hinweg und darüber hinauskragend erstreckt ist und durch einen gestuften Bereich (6.1.a) an der Ausnehmung (6.1) des gegenüberliegenden Elementes (6) axial abgeknickt ist in einen Bereich hinein, der radial außen vom Vorsprung (1.2), insbesondere auch axial hinter den Vorsprung (1.2) zurücktretend angeordnet ist und/oder der innere Dichtungsring (5b) in radialer Richtung nach innen über den Vorsprung (2a.2) hinweg und darüber hinauskragend erstreckt ist und durch einen gestuften Bereich an der Ausnehmung (6c.1) des gegenüberliegenden Elementes (6c) axial abgeknickt ist in einen Bereich hinein, der radial innen vom Vorsprung (2a.2), insbesondere auch axial hinter den Vorsprung (2a.2) zurücktretend angeordnet ist.

7. Verfahren zur Herstellung einer Vorrichtung für den Stoffaustausch zwischen einem ersten Medium, insbesondere Blut, und einem zweiten Medium, insbesondere einem Gas/Gasgemisch, vorzugsweise zur Herstellung einer Vorrichtung nach einem der vorherigen Ansprüche, wobei in ein Gehäuseelement (1) der Vorrichtung ein einen Wickelkern (2a) umgebendes Hohlfaserpaket (2), mit zwischen den axialen Enden des Gehäuseelementes (1) erstreckten stoffpermeablen Hohlfasern angeordnet wird, die nach Verschließen des Gehäuseelementes (1) an wenigstens einem seiner axialen Enden, vorzugsweise an beiden Enden, mit einem Vergussdeckel (3) an ihrem vom Vergussdeckel (3) überdeckten axialen Endbereich zumindest untereinander mit einer Vergussmasse (4) vergossen werden, insbesondere in einer Zentrifuge, nach dem Vergießen der Vergussdeckel (3) entfernt, die vergossenen Hohlfasern an ihren axialen Enden eröffnet werden und das Gehäuseelement (1) mit einem Deckelelement (6) dicht verschlossen wird, **dadurch gekennzeichnet, dass** aus der Vergussmasse (4) gleichzeitig mit dem Vergiessen der Hohlfasern untereinander ein das Hohlfaserpaket (2) radial außen umgebender Dichtungsring (5a) erstellt wird durch Hineinfließen der Vergussmasse (4) in einen zwischen den Stirnflächen von Gehäuseelement (1) und Vergussdeckel (3) ausgebildeten Ringspalt (1.1, 3.1), insbesondere äußeren Ringspalt (1.1, 3.1) und aus der Vergussmasse (4) gleichzeitig mit dem Vergiessen der Hohlfasern untereinander am inneren Umfang des Hohlfaserpakets (2) ein innerer Dichtungsring (5b) erstellt wird durch Hineinfließen der Vergussmasse (4) in einen zwischen den Stirnflächen von dem Wickelkern (2a), der das Hohlfaserpaket (2) trägt und einem den Wickelkern (2a) abdeckenden Abdeckelement (3b) im Vergussdeckel (3) ausgebildeten Ringspalt (2a.1, 3b.1), insbesondere inneren Ringspalt (2a.1, 3b.1).

8. Verfahren nach Anspruch 7, **dadurch gekennzeichnet, dass** der Ringspalt (1.1, 3.1), insbesondere äußere Ringspalt (1.1, 3.1), definiert wird durch eine axial und radial nach innen offene Ausnehmung (1.1, 3.1) in der Stirnfläche vom Gehäuseelement (1) und/oder vom Vergussdeckel (3) und/oder der Ringspalt (2a.1, 3b.1), insbesondere innere Ringspalt (2a.1, 3b.1), definiert wird durch eine axial und radial nach außen offene Ausnehmung (2a.1, 3b.1) in der Stirnfläche vom Wickelkern (2a) und/oder vom Abdeckelement (3b) im Vergussdeckel (3).

9. Verfahren nach einem der vorherigen Ansprüche 7 oder 8, **dadurch gekennzeichnet, dass** mit dem Eröffnen der Hohlfaserenden, insbesondere durch Abschneiden von vergossenen Hohlfaserenden oder auch durch Abtrennen von herstellerseitig geschlossenen Hohlfaserenden, auch der äußere Dichtungsring (5a) und/oder der innere Dichtungsring (5b) vom verklebten Hohlfaserpaket (2) getrennt wird, insbesondere der äußere Dichtungsring (5a) in einer zumindest in axialer Richtung offenen Ausnehmung in der Stirnfläche des Gehäuseelementes (1) verbleibt und durch Aufsetzen des Deckelelementes (6) zwischen den Stirnflächen von Deckelelement (6) und Gehäuseelement (1) komprimiert wird und/oder der innere Dichtungsring (5b) in einer zumindest in axialer Richtung offenen Ausnehmung in der Stirnfläche des Wickelkerns (2a) verbleibt und durch Aufsetzen des Stopfens (6c) zwischen den Stirnflächen von Stopfen (6c) und Wickelkern (2a) komprimiert wird.

10. Verfahren nach einem der vorherigen Ansprüche 7 oder 8, **dadurch gekennzeichnet, dass** nach dem Eröffnen der Hohlfaserenden, insbesondere durch Abschneiden von vergossenen Hohlfaserenden oder auch durch Abtrennen von herstellerseitig geschlossenen Hohlfaserenden, der äußere Dichtungsring (5a) und/oder der innere Dichtungsring (5b) stoffschlüssig / einstückig am Hohlfaserpaket (2) befestigt bleibt, insbesondere der äußere Dichtungsring (5a) durch Aufsetzen des Deckelelementes (6) zwischen den Stirnflächen von Deckelelement (6) und Gehäuseelement (1) komprimiert wird und/oder der innere Dichtungsring (5b) durch Aufsetzen des Stopfens (6c) zwischen den Stirnflächen von Stopfen (6c) und Wickelkern (2a) komprimiert wird.

11. Verfahren nach einem der vorherigen Ansprüche 7 bis 10, **dadurch gekennzeichnet, dass** durch die Formgebung des zwischen Vergussdeckel (3) und Gehäuseelement (1) definierten Ringspaltes (1.1, 3.1) ein äußerer Dichtungsring (5a) ausgebildet wird, der nach Entfernen des Vergussdeckels (3)
a. in einer radial innen in der Stirnfläche des Gehäuseelementes (1) angeordneten Ausnehmung (1.1) einliegt, vorzugsweise hierbei in axialer Richtung über den die Ausnehmung (1.1) umgebenden Stirnflächenbereich des Gehäuseelementes (1) übersteht, und durch Aufsetzen des Deckelelementes (6) in die Ausnehmung (1.1) hinein komprimiert wird, insbesondere durch einen radial innen an der Stirnfläche des Deckelelementes (6) angeordneten axialen Vorsprung (6.2), oder
b. auf einem in der radialen Erstreckung ohne Ausnehmung ausgebildeten, planen Oberflächenbereich der Stirnfläche des Gehäuseelementes (1) in einer vorbestimmten Höhe aufliegt und durch Aufsetzen des Deckelelementes (6) in einer axial und radial nach innen offenen Ausnehmung (6.1) in der Stirnfläche des Deckelelementes (6) mit einer Tiefe kleiner als die vorbestimmte Höhe komprimiert wird, oder
c. einen radial innenliegenden axialen Vorsprung (1.2) in der Stirnfläche des Gehäuseelementes (1) überdeckt und in radialer Richtung nach außen überkragt und beim Aufsetzen des Deckelelementes (6) durch eine den Vorsprung (1.2) umgreifende Ausnehmung (6.1) in der Stirnfläche des Deckelelementes (6) komprimiert und in axialer Richtung abgeknickt wird.

12. Verfahren nach einem der vorherigen Ansprüche 7 bis 11, **dadurch gekennzeichnet, dass** durch die Formgebung des zwischen dem Abdeckelement (3b) im Vergussdeckel (3) und Wickelkern (2a) definierten Ringspaltes (2a.1, 3b.1) ein innerer Dichtungsring (5b) ausgebildet wird, der nach Entfernen des Abdeckelementes (3b) und des Vergussdeckels (3)
a. in einer radial außen in der Stirnfläche des Wickelkerns (2a) angeordneten Ausnehmung (2a.1) einliegt, vorzugsweise hierbei in axialer Richtung über den radial innen von der Ausnehmung (2a.1) liegenden Stirnflächenbereich des Wickelkerns (2a) übersteht, und durch Aufsetzen des Stopfens (6c) in die Ausnehmung (2a.1) hinein komprimiert wird, insbesondere durch einen radial außen an der Stirnfläche des Stopfens (6c) angeordneten axialen Vorsprung (6c.2), oder
b. auf einem in der radialen Erstreckung ohne Ausnehmung ausgebildeten, planen Oberflächenbereich der Stirnfläche des Wickelkerns (2a) in einer vorbestimmten Höhe aufliegt und durch Aufsetzen des Stopfens (6c) in einer axial und radial nach außen offenen Ausnehmung (6c.1) in der Stirnfläche des Stopfens (6c) mit einer Tiefe kleiner als die vorbestimmte Höhe komprimiert wird, oder
c. einen radial außenliegenden axialen Vorsprung (2a.2) in der Stirnfläche des Wickelkerns (2a) überdeckt und in radialer Richtung nach innen überkragt und beim Aufsetzen des Stopfens (6c) durch eine den Vorsprung umgreifende Ausnehmung (6c.1) in der Stirnfläche des Stopfens (6c) komprimiert und in axialer Richtung abgeknickt wird.

## Claims

1. Device for substance exchange between a first medium, in particular blood, and a second medium, in particular a gas/gas mixture, comprising a housing element (1) in which there is arranged a hollow-fibre bundle (2) containing substance-permeable hollow fibres, which extend axially between the axial ends of the housing element (1), are wound on a winding core and at their respective axial end region are potted with a potting compound (4), at least among one another, and around which the first medium can flow and through which the second medium can flow, wherein the housing element (1), at at least one of the axial ends, preferably at both axial ends, is closed by a cover element (6), in particular one which has a media connection (6.1) in fluidic communication with the axially open hollow-fibre ends, **characterized in that**, at at least one of the axial ends of the housing element (1), preferably at both ends, an outer sealing ring (5a) surrounding the hollow-fibre bundle (2) is formed from the potting compound (4) arranged between the hollow fibres, which outer sealing ring extends in the radial direction at least in part between the axial end faces of the housing element (1) and of the cover element (6) and is compressed between these, and at at least one of the axial ends of the winding core (2a) which carries the hollow fibres (2), preferably at both ends,
an inner sealing ring (5b) arranged on the inner circumference of the hollow-fibre bundle (2) is formed from the potting compound (4) arranged between the hollow fibres, which inner sealing ring extends in the radial direction at least in part between the axial end faces of the winding core (2a) and of a plug (6c) in the cover element (6) and is compressed between these.

2. Device according to Claim 1, **characterized in that** the outer and/or inner sealing ring (5a, 5b) is in one piece with/cohesively bonded to the potting compound (4) between the hollow fibres.

3. Device according to one of the preceding claims, **characterized in that** the potting compound (4) is formed by an elastomer, preferably with a hardness of less than Shore A 100, preferably less than Shore A 60, more preferably less than Shore A 30, in particular with the potting compound (4) being formed from a silicone/silicone rubber.

4. Device according to one of the preceding claims, **characterized in that** the axial end face of the housing element (1) and/or of the cover element (6) has, radially inwardly, an axially and radially inwardly open recess (1.1, 6.1), in which the outer sealing ring (5a) lies compressed, and/or the axial end face of the winding core (2a) and/or of the plug (6c) in the cover element (6) has, radially outwardly, an axially and radially outwardly open recess (1.1, 6.1), in which the inner sealing ring (5b) lies compressed.

5. Device according to Claim 4, **characterized in that** an axial, preferably annular, projection (6.2, 1.2) is arranged radially inwardly on one of the end faces of the housing element (1) or cover element (6) and lies at least in part in the axially open recess (1.1 , 6.1) in the end face of the opposite element (1, 6), or an axial, preferably annular, projection (6c.2, 2a.2) is arranged radially outwardly on one of the end faces of the winding core (2a) or of the plug (6c) in the cover element (6) and lies at least in part in the axially open recess (2a.1, 6c.1) in the end face of the opposite element (6c, 2a).

6. Device according to Claim 5, **characterized in that** the outer sealing ring (5a) is extended in the radial direction outwardly beyond the projection (1.2) and overhangs it and, by a stepped region (6.1.a) at the recess (6.1) of the opposite element (6), is bent axially into a region which is arranged set back radially outwardly from the projection (1.2), in particular also axially behind the projection (1.2), and/or the inner sealing ring (5b) is extended in the radial direction inwardly beyond the projection (2a.2) and overhangs it and, by a stepped region at the recess (6c.1) of the opposite element (6c), is bent axially into a region which is arranged set back radially inwardly from the projection (2a.2), in particular also axially behind the projection (2a.2).

7. Method for production of a device for substance exchange between a first medium, in particular blood, and a second medium, in particular a gas/gas mixture, preferably for production of a device according to one of the preceding claims, wherein a hollow-fibre bundle (2) surrounding a winding core (2a), with substance-permeable hollow fibres extending between the axial ends of the housing element (1), is arranged in a housing element (1) of the device, which hollow fibres, after closure of the housing element (1) at at least one of its axial ends, preferably at both ends, are potted with a potting compound (4), at least among one another, with a potting cover (3) at their axial end region covered by the potting cover (3), in particular in a centrifuge, and, after the potting, the potting cover (3) is removed, the potted hollow fibres are opened at their axial ends, and the housing element (1) is tightly sealed with a cover element (6), **characterized in that**, simultaneously with the potting of the hollow fibres among one another, a sealing ring (5a) surrounding the hollow-fibre bundle (2) radially on the outside is created from the potting compound (4), by the potting compound (4) flowing into an annular gap (1.1, 3.1) formed between the end faces of housing element (1) and potting cover (3), in particular an outer annular gap (1.1, 3.1), and, simultaneously with the potting of the hollow fibres among one another at the inner circumference of the hollow-fibre bundle (2), an inner sealing ring (5b) is created from the potting compound (4), by the potting compound (4) flowing into an annular gap (2a.1, 3b.1), in particular an inner annular gap (2a.1, 3b.1), formed between the end faces of the winding core (2a), which carries the hollow-fibre bundle (2), and
a cover element (3b) in the potting cover (3) covering the winding core (2a).

8. Method according to Claim 7, **characterized in that** the annular gap (1.1, 3.1), in particular the outer annular gap (1.1, 3.1), is defined by an axially and radially inwardly open recess (1.1, 3.1) in the end face of the housing element (1) and/or of the potting cover (3), and/or the annular gap (2a.1, 3b.1), in particular the inner annular gap (2a.1, 3b.1), is defined by an axially and radially outwardly open recess (2a.1, 3b.1) in the end face of the winding core (2a) and/or of the cover element (3b) in the potting cover (3).

9. Method according to either of preceding Claims 7 and 8, **characterized in that**, with the opening of the hollow-fibre ends, in particular by cutting off potted hollow-fibre ends or also by separating hollow-fibre ends closed by the manufacturer, the outer sealing ring (5a) and/or the inner sealing ring (5b) is also separated from the bonded hollow-fibre bundle (2), in particular the outer sealing ring (5a) remains in a recess, open at least in the axial direction, in the end face of the housing element (1), and, by placement of the cover element (6), is compressed between the end faces of cover element (6) and housing element (1), and/or the inner sealing ring (5b) remains in a recess, open at least in the axial direction, in the end face of the winding core (2a), and, by placement of the plug (6c), is compressed between the end faces of plug (6c) and winding core (2a).

10. Method according to either of preceding Claims 7 and 8, **characterized in that**, after the opening of the hollow-fibre ends, in particular by cutting off potted hollow fibre ends or also by separating hollow-fibre ends closed by the manufacturer, the outer sealing ring (5a) and/or the inner sealing ring (5b) remains attached to the hollow-fibre bundle (2) by cohesive bonding/integral connection, in particular the outer sealing ring (5a), by placement of the cover element (6), is compressed between the end faces of cover element (6) and housing element (1), and/or the inner sealing ring (5b), by placement of the plug (6c), is compressed between the end faces of plug (6c) and winding core (2a).

11. Method according to one of preceding Claims 7 to 10, **characterized in that** the shape of the annular gap (1.1, 3.1) defined between potting cover (3) and housing element (1) forms an outer sealing ring (5a) which, after removal of the potting cover (3),
a. lies in a recess (1.1) arranged radially inwardly in the end face of the housing element (1), preferably protruding in the axial direction beyond the end face region of the housing element (1) surrounding the recess (1.1), and, by placement of the cover element (6), is compressed into the recess (1.1), in particular by an axial projection (6.2) arranged radially inwardly on the end face of the cover element (6), or
b. rests at a predetermined height on a planar surface region of the end face of the housing element (1) that is formed in the radial extent without a recess and, by placement of the cover element (6), is compressed in an axially and radially inwardly open recess (6.1) in the end face of the cover element (6), to a depth less than the predetermined height, or
c. covers a radially inner axial projection (1.2) in the end face of the housing element (1) and protrudes outward in the radial direction and, upon placement of the cover element (6), is compressed by a recess (6.1) surrounding the projection (1.2) in the end face of the cover element (6) and bent in the axial direction.

12. Method according to one of preceding Claims 7 to 11, **characterized in that** the shape of the annular gap (2a.1, 3b.1) defined between the cover element (3b) in the potting cover (3) and the winding core (2a) forms an inner sealing ring (5b) which, after removal of the cover element (3b) and of the potting cover (3),
a. lies in a recess (2a.1) arranged radially outwardly in the end face of the winding core (2a), preferably protruding in the axial direction beyond the end face region of the winding core (2a) lying radially inward from the recess (2a.1), and, by placement of the plug (6c), is compressed into the recess (2a.1), in particular by an axial projection (6c.2) arranged radially outwardly on the end face of the plug (6c), or
b. rests at a predetermined height on a planar surface region of the end face of the winding core (2a) that is formed in the radial extent without a recess and, by placement of the plug (6c), is compressed in an axially and radially outwardly open recess (6c.1) in the end face of the plug (6c), to a depth less than the predetermined height, or
c. covers a radially outer axial projection (2a.2) in the end face of the winding core (2a) and protrudes inward in the radial direction and, upon placement of the plug (6c), is compressed by a recess (6c.1) surrounding the projection in the end face of the plug (6c) and bent in the axial direction.

## Revendications

1. Dispositif d'échange de matière entre un premier milieu, en particulier du sang, et un deuxième milieu, en particulier un gaz/un mélange de gaz, comprenant un élément de boîtier (1), dans lequel est disposé un paquet de fibres creuses (2) avec des fibres creuses perméables à la matière s'étendant axialement entre les extrémités axiales de l'élément de boîtier (1) et enroulées sur un noyau d'enroulement, qui sont scellées entre elles avec une masse de scellement (4) sur leur zone d'extrémité axiale respective et qui peuvent être entourées par le premier milieu et peuvent être traversées par le deuxième milieu, l'élément de boîtier (1) étant fermé sur au moins une des extrémités axiales, de préférence sur les deux extrémités axiales, par un élément de couvercle (6), qui comporte en particulier un raccord de milieu (6.1), qui est en communication fluidique avec les extrémités de fibres creuses axialement ouvertes, **caractérisé en ce qu'**est formée, sur au moins une des extrémités axiales de l'élément de boîtier (1), de préférence sur les deux, à partir de la masse de scellement (4) disposée entre les fibres creuses, une bague d'étanchéité extérieure (5a) entourant le paquet de fibres creuses (2), qui s'étend dans une direction radiale, au moins par endroits, entre les faces frontales axiales de l'élément de boîtier (1) et de l'élément de couvercle (6) et est comprimée entre celles-ci et sur au moins une des extrémités axiales du noyau d'enroulement (2a), qui supporte les fibres creuses (2),
de préférence sur les deux,
est formée , à partir de la masse de scellement (4) disposée entre les fibres creuses, une bague d'étanchéité intérieure (5b) disposée sur la périphérie intérieure du paquet de fibres creuses (2), qui s'étend dans une direction radiale au moins par endroits entre les faces frontales axiales du noyau d'enroulement (2a) et d'un bouchon (6c) dans l'élément de couvercle (6) et est comprimée entre celles-ci.

2. Dispositif selon la revendication 1, **caractérisé en ce que** la bague d'étanchéité extérieure et/ou intérieure (5a, 5b) est reliée d'un seul tenant/par liaison de matière à la masse de scellement(4).

3. Dispositif selon l'une des revendications précédentes, **caractérisé en ce que** la masse de scellement (4) est formée par un élastomère, de préférence avec une dureté Shore A inférieure à 100, de préférence une dureté Shore A inférieure à 60, par ailleurs de manière préférée avec une dureté Shore A inférieure à 30, en particulier la masse de scellement (4) est formée à partir d'un silicone/d'un caoutchouc de silicone.

4. Dispositif selon l'une des revendications précédentes, **caractérisé en ce que** la face frontale axiale de l'élément de boîtier (1) et/ou de l'élément de couvercle (6) comporte radialement à l'intérieur un évidement (1.1, 6.1) ouvert axialement et radialement vers l'intérieur, dans lequel la bague d'étanchéité extérieure (5a) est comprimée et/ou la face frontale axiale du noyau d'enroulement (2a) et/ou du bouchon (6c) de l'élément de couvercle (6) comporte radialement à l'extérieur un évidement (1.1, 6.1) axialement et radialement ouvert vers l'extérieur, dans lequel la bague d'étanchéité intérieure (5b) est comprimée.

5. Dispositif selon la revendication 4, **caractérisé en ce qu'**est disposée radialement à l'intérieur, sur une des faces frontales de l'élément de boîtier (1) ou de l'élément de couvercle (6), une saillie (6.2, 1.2) axiale, de préférence annulaire, qui se trouve au moins par endroits dans l'évidement (1.1, 6.1) axialement ouvert dans la face frontale de l'élément opposé (1, 6) ou est disposée radialement à l'extérieur, sur une des faces frontales du noyau d'enroulement (2a) ou du bouchon (6c), dans l'élément de couvercle (6), une saillie (6c.2, 2a.2) axiale, de préférence annulaire, qui se trouve au moins par endroits dans l'évidement (2a.1, 6c.1) axialement ouvert dans la face frontale de l'élément opposé (6c, 2a).

6. Dispositif selon la revendication 5, **caractérisé en ce que** la bague d'étanchéité extérieure (5a) s'étend dans la direction radiale vers l'extérieur au-delà de la saillie (1.2) et au-delà en porte-à-faux et est pliée axialement par une zone étagée (6.1.a) sur l'évidement (6.1) de l'élément opposé (6), à l'intérieur d'une zone, qui est disposée en retrait radialement à l'extérieur de la saillie (1.2), en particulier également axialement derrière la saillie (1.2) et/ou la bague d'étanchéité intérieure (5b) s'étend dans la direction radiale vers l'intérieur au-delà de la saillie (2a.2) et au-delà en porte-à-faux et est pliée axialement par une zone étagée sur l'évidement (6c.1) de l'élément opposé (6c), à l'intérieur d'une zone, qui est disposée en retrait radialement à l'intérieur de la saillie (2a.2), en particulier également axialement derrière la saillie (2a.2).

7. Procédé de fabrication d'un dispositif d'échange de matière entre un premier milieu, en particulier du sang, et un deuxième milieu, en particulier un gaz/un mélange de gaz, de préférence de fabrication d'un dispositif selon l'une des revendications précédentes, un paquet de fibres creuses (2) entourant un noyau d'enroulement (2a) avec des fibres creuses perméable à la matière s'étendant entre les extrémités axiales de l'élément de boîtier (1) étant disposé dans un élément de boîtier (1) du dispositif, lesquelles sont scellées au moins entre elles avec une masse de scellement (4) sur leur zone d'extrémité axiale recouverte par le couvercle de scellement (3) avec un couvercle de scellement (3), après la fermeture de l'élément de boîtier (1) sur au moins une de ses extrémités axiales, de préférence sur les deux extrémités, en particulier dans une centrifugeuse, le couvercle de scellement (3) étant retiré après le scellement, les fibres creuses scellées étant ouvertes sur leurs extrémités axiales et l'élément de boîtier (1) étant scellé de manière étanche avec un élément de couvercle (6), **caractérisé en ce qu'**une bague d'étanchéité (5a) entourant radialement à l'extérieur le paquet de fibres creuses (2) est créée à partir de la masse de scellement (4) simultanément avec le scellement des fibres creuses entre elles en introduisant la masse de scellement (4) dans une fente annulaire (1.1, 3.1) formée entre les faces frontales de l'élément de boîtier (1) et du couvercle de scellement (3), en particulier une fente annulaire extérieure (1.1, 3.1) et une bague d'étanchéité intérieure (5b) étant créée à partir de la masse de scellement (4) simultanément avec le scellement des fibres creuses entre elles sur la périphérie intérieure du paquet de fibres creuses (2) en introduisant la masse de scellement (4) dans une fente annulaire (2a.1, 3b.1) formée entre les faces frontales du noyau d'enroulement (2a), qui supporte le paquet de fibres creuses (2),
et
un élément de recouvrement (3b) recouvrant le noyau d'enroulement (2a) dans le couvercle de scellement (3), en particulier une fente annulaire intérieure (2a.1, 3b.1).

8. Procédé selon la revendication 7, **caractérisé en ce que** la fente annulaire (1.1, 3.1), en particulier la fente annulaire extérieure (1.1, 3.1), est définie par un évidement (1.1, 3.1) axialement et radialement ouvert vers l'intérieur dans la face frontale de l'élément de boîtier (1) et/ou du couvercle de scellement (3) et/ou la fente annulaire (2a.1, 3b.1), en particulier la fente annulaire intérieure (2a.1, 3b.1), est définie par un évidement (2a.1, 3b.1) axialement et radialement ouvert vers l'extérieur dans la face frontale du noyau d'enroulement (2a) et/ou de l'élément de recouvrement (3b) dans le couvercle de scellement (3).

9. Procédé selon l'une des revendications 7 ou 8 précédentes, **caractérisé en ce que** l'ouverture des extrémités de fibre creuse, en particulier en coupant les extrémités de fibre creuse scellées ou en séparant les extrémités de fibre creuse fermées par le fabricant, permet de séparer également la bague d'étanchéité extérieure (5a) et/ou la bague d'étanchéité intérieure (5b) du paquet de fibres creuses (2) collées, en particulier la bague d'étanchéité extérieure (5a) reste dans la face frontale de l'élément de boîtier (1) dans un évidement ouvert au moins dans la direction axiale et est comprimée en plaçant l'élément de couvercle (6) entre les faces frontales de l'élément de couvercle (6) et de l'élément de boîtier (1) et/ou la bague d'étanchéité intérieure (5b) reste dans la face frontale du noyau d'enroulement (2a) dans un évidement ouvert au moins dans la direction axiale et est comprimée en plaçant le bouchon (6c) entre les faces frontales du bouchon (6c) et du noyau d'enroulement (2a).

10. Procédé selon l'une des revendications 7 ou 8 précédentes, **caractérisé en ce que**, après l'ouverture des extrémités de fibre creuse, en particulier en coupant les extrémités de fibre creuse scellées ou en séparant également des extrémités de fibre creuse scellées par le fabricant, la bague d'étanchéité extérieure (5a) et/ou la bague d'étanchéité intérieure (5b) restent fixées par liaison de matière/d'un seul tenant sur le paquet de fibres creuses (2), en particulier, la bague d'étanchéité extérieure (5a) est comprimée en plaçant l'élément de couvercle (6) entre les faces frontales de l'élément de couvercle (6) et de l'élément de boîtier (1) et/ou la bague d'étanchéité intérieure (5b) est comprimée en plaçant le bouchon (6c) entre les faces frontales du bouchon (6c) et du noyau d'enroulement (2a).

11. Procédé selon l'une des revendications précédentes 7 à 10, **caractérisé en ce que** la forme de la fente annulaire (1.1, 3.1) définie entre le couvercle de scellement (3) et l'élément de boîtier (1) permet de former une bague d'étanchéité extérieure (5a) qui, après le retrait du couvercle de scellement (3),
a. se trouve dans un évidement (1.1) disposé radialement à l'intérieur dans la face frontale de l'élément de boîtier (1), de préférence dépasse dans la direction axiale de la zone de face frontale, entourant l'évidement (1.1), de l'élément de boîtier (1), et est comprimée en plaçant l'élément de couvercle (6) dans l'évidement (1.1), en particulier par une saillie axiale (6.2) disposée radialement à l'intérieur sur la face frontale de l'élément de couvercle (6), ou
b. repose à une hauteur prédéfinie sur une zone de surface plane, formée sans évidement dans l'extension radiale, de la face frontale de l'élément de boîtier (1) et est comprimée à une profondeur inférieure à la hauteur prédéfinie en plaçant l'élément de couvercle (6) dans un évidement (6.1) axialement et radialement ouvert vers l'intérieur dans la face frontale de l'élément de couvercle (6), ou
c. recouvre une saillie axiale (1.2) située à l'intérieur radialement dans la face frontale de l'élément de boîtier (1) et est en porte-à-faux vers l'extérieur dans la direction radiale et est comprimée dans la face frontale de l'élément de recouvrement (6) par un évidement (6.1) entourant la saillie (1.2) en plaçant l'élément de couvercle (6) et est pliée dans la direction axiale.

12. Procédé selon l'une des revendications précédentes 7 à 11, **caractérisé en ce que** la forme de la fente annulaire (2a.1, 3b.1) définie entre l'élément de recouvrement (3b) dans le couvercle de scellement (3) et le noyau d'enroulement (2a) permet de former une bague d'étanchéité intérieure (5b) qui, après le retrait de l'élément de recouvrement (3b) et du couvercle de scellement (3)
a. se trouve dans un évidement (2a.1) disposé radialement à l'extérieur dans la face frontale du noyau d'enroulement (2a), de préférence dépasse dans la direction axiale, de la zone de face frontale du noyau d'enroulement (2a) située radialement à l'intérieur de l'évidement (2a.1), et est comprimée en plaçant le bouchon (6c) à l'intérieur de l'évidement (2a.1), en particulier par une saillie axiale (6c.2) disposée radialement à l'extérieur sur la face frontale du bouchon (6c), ou
b. repose à une hauteur prédéfinie sur une zone de surface plane, formée sans évidement dans l'extension radiale, de la face frontale du noyau d'enroulement (2a) et est comprimée à une profondeur inférieure à la hauteur prédéfinie en plaçant le bouchon (6c) dans un évidement (6c.1) axialement et radialement ouvert vers l'extérieur dans la face frontale du bouchon (6c), ou
c. recouvre une saillie axiale (2a.2) située radialement à l'extérieur sur la face frontale du noyau d'enroulement (2a) et la dépasse en porte-à-faux dans la direction radiale vers l'intérieur et, lors du placement du bouchon (6c), est comprimée et pliée dans la direction axiale par un évidement (6c.1) entourant la saillie dans la face frontale du bouchon (6c).
